(19) Europäisches Patentamt
European Patent Office
Office européen des brevets

(11) Publication number: 0 330 432
A1

(12) **EUROPEAN PATENT APPLICATION**

(21) Application number: 89301678.2

(22) Date of filing: 22.02.89

(51) Int. Cl.4: **C 07 D 203/14**
C 12 P 17/10, A 61 K 31/395,
A 61 K 37/50, C 07 K 15/00

(30) Priority: 22.02.88 GB 8804068

(43) Date of publication of application:
30.08.89 Bulletin 89/35

(84) Designated Contracting States: ES GR

(71) Applicant: **Roberts, John J.**
**The Department of Molecular Pharmacology, Institute of Cancer Research Clifton Avenue**
**Sutton Surrey SM2 2PX (GB)**

**Knox, Richard J.**
**The Department of Molecular Pharmacology Institute of Cancer Research Clifton Avenue**
**Sutton Surrey SM2 2PX (GB)**

**Friedlos, Frank**
**The Department of Molecular Pharmacology Institute of Cancer Research Clifton Avenue**
**Sutton Surrey SM2 2PX (GB)**

(72) Inventor: **Roberts, John J.**
**The Department of Molecular Pharmacology, Institute of Cancer Research Clifton Avenue**
**Sutton Surrey SM2 2PX (GB)**

**Knox, Richard J.**
**The Department of Molecular Pharmacology Institute of Cancer Research Clifton Avenue**
**Sutton Surrey SM2 2PX (GB)**

**Friedlos, Frank**
**The Department of Molecular Pharmacology Institute of Cancer Research Clifton Avenue**
**Sutton Surrey SM2 2PX (GB)**

(74) Representative: **Goldin, Douglas Michael et al**
**J.A. KEMP & CO. 14, South Square Gray's Inn**
**London WC1R 5EU (GB)**

(54) **Improvements relating to the control of neoplastic tissue growth.**

(57) Compounds of the general formula:

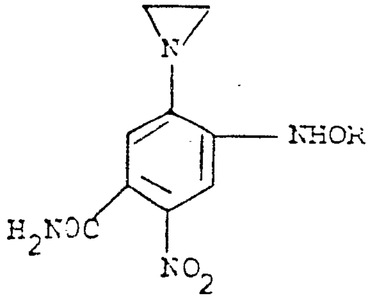

wherein R is H, an acyl group or a hydrocarbyl group containing up to 6 carbon atoms, have cytotoxic activity against several tumours. The 4-hydroxylamino compound can be obtained directly from the corresponding 2,4-dinitro compound by selective chemical reduction or by the action of a nitroreductase obtainable from Walker tumour cells. The 2,4-dinitro compound is substantially devoid of cytotoxic activity except in relation to Walker tumour cells and hence can act as a prodrug in regimes where a prodrug is localised in vivo in the regions of a tumour to be treated and is then activated to the cytotoxic 4-hydroxylamino compound under the influence of the enzyme.

EP 0 330 432 A1

## Description

THIS INVENTION relates to the control of neoplastic tissue growth and is particularly concerned with the provision of new anti-tumour agents and with enzymes capable of converting anti-tumour pro-drugs into anti-tumour agents.

The alkylating agent 5-(aziridin-1-yl)-2,4-dinitrobenzamide (hereinafter designated CB 1954) has been known, almost for 20 years, as an interesting experimental compound of unique selectivity. Although CB 1954 is structurally quite closely related to numerous other known alkylating agents which have a relatively broad range of activity, CB 1954 exhibits considerable activity against the Walker tumour cells, in vivo or in vitro, but was thought to be virtually inactive against other tumours.

We believe that we have now found an explanation for the selectivity of CB 1954 in that CB 1954 is not an anti-tumour agent per se but is in fact a pro-drug for an anti-tumour agent generated from CB 1954 by a nitroreductase enzyme found in the Walker cell. We have examined both this Walker cell enzyme and the nature of the anti-tumour agent generated from CB 1954 by the enzyme and the present invention is directed to both aspects of this investigation.

Our initial characterisation of the enzyme we isolated from CB1954 was of an enzyme of molecular weight approximately 33,500 daltons having the following characteristics:

1. It has the ability to reduce CB 1954 (in the presence of NADH) to 5-(aziridin-1-yl)-4-hydroxylamino-2-nitrobenzamide. This reduction takes place at the same rate under either air or nitrogen.

2. Its absorption spectrum has a peak at 450 nm giving the protein a characteristic yellow colour implying a flavin cofactor.

3. On heating at 56°C for 20 minutes, the flavin cofactor could be separated from the protein and isolated by ultrafiltration. Spectral data suggested that the flavin was flavin adenine dinucleotide (FAD) and this was confirmed by the method of Pietta et al, J. Chromatog. 229, 445-449 (1982).

4. The enzyme does not reduce 2,4-dinitrophenol but will reduce 2,6-dichlorophenolindophenol with either NADH or NAPH as cofactors.

5. The enzyme does not readily reduce the 2-nitro group of CB 1954.

When we sequenced the isolated pure enzyme we found it to have a very substantial sequence homology with the NAD(P)H dehydrogenase (quinone) now classified as EC.1.6.99.2, see Robertson et al. J. Biol. Chem. 261, 15794-15799 (1986). The extent of the sequence homology, together with other tests, leads us to conclude that the 33.5 Kd enzyme that we isolated from Walker tumour cells, and that we previously thought to be a new enzyme, is in fact the same as EC.1.6.99.2 of Robertson et al. However, Robertson et al(1986) does not disclose the existence of this enzyme in Walker tumour cells or its selectivity to CB 1954 conversion that we found for it.

The fact that the new enzyme can convert CB 1954 into an anti-tumour compound identified as the 4-hydroxylamino derivative has been confirmed by the unambiguous synthesis of 5-(aziridin-1-yl)-4-hydroxylamino-2-nitrobenzamide which was shown to be identical with the product obtained by Walker cell enzyme conversion of CB 1954.

The enzyme used in the invention can be isolated from the cells of the Walker tumour by extracting the Walker cells and subjecting the extract to sucessive gel filtration and ion exchange high performance liquid chromatography.

Given the relatively small size of the enzyme, as an alternative to recovery from natural sources, the enzyme could be prepared synthetically, by conventional peptide synthesis, e.g. solid phase Merrifield synthesis, or by recombinant DNA techniques by expressing, in a suitable host cell, DNA encoding the enzyme. DNA encoding the enzyme can be obtained by preparing a synthetic polynucleotide encoding the enzyme. Alternatively, conventional techniques could be used using reverse transcriptase with messenger RNA isolated from the Walker cells.

The enzyme is of particular interest in that it can be utilised to increase the area of applicability of CB 1954 which now has potential clinical application as a pro-drug in view of its relatively low cytotoxicity but, under the influence of the enzyme, is converted into its much more toxic 4-hydroxylamino derivative. As a result of our discovery, a further investigation has been made into the activity of CB 1954 against various tumour cells and we found activity (as CB 1954 or as a metabolite thereof) against various ovarian, renal, breast and lung tumour cells.

The utilisation of enzymes in conjunction with anti-tumour pro-drugs is now a technique of increasing interest in the field of cancer chemotherapy, interest centering especially upon anti-tumour pro-drugs where the cytotoxicity ratio between the pro-drug and the drug is sufficiently great. The discovery of an enzyme capable of converting a monofunctional alkylating agent such as CB 1954 into a difunctional agent having a cytotoxicity considerably greater than that of CB 1954 itself is clearly a significant breakthrough in cancer chemotherapy.

The availability of the enzyme is of value not only in that it can be used to convert a cytotoxic pro-drug into the cytotoxic drug as a general technique but, by using existing techniques, it is possible to conjugate the enzyme to an antibody, particularly a monoclonal antibody, that will bind with tumour associated antigens. In this way, the enzyme can be directed to the tumour site and bring about a localisation of enzyme in the region of the tumour. Subsequent administration of the cytotoxic pro-drug will ensure that there is a localisation of cytotoxic drug in the region of the tumour with a correspondingly lower concentration of cytotoxic drug and higher concentration of the less toxic pro-drug in

other parts of the body. The enzyme is also of interest in that antibodies, particularly monoclonal antibodies, to the enzyme can be generated by conventional techniques and assist in the detection of CB 1954 sensitive tumours.

In accordance with the present invention, we provide a new anti-tumour compound which is 5-(aziridin-1-yl)-4-hydroxylamino-2-nitrobenzamide and its derivatives. By derivatives, we mean derivatives of the hydroxy group e.g., ester and ether derivatives.

The ester derivatives of the present invention are essentially esters of the hydroxylamine with lower alkanoic acids, e.g. those containing from 1 to 6 carbon atoms and in this category, attention focusses on the saturated alkanoic acids, particularly those containing a straight chain of 2 to 4 carbon atoms such as the acetate and propionate ester. Esters with other acids, e.g., phosphate and sulphate esters are also within the scope of the invention. Ether derivatives of the hydroxylamine are preferably those containing a hydrocarbyl ether group of 1 to 6 carbon atoms, particularly where the hydrocarbyl group is a $C_1$-$C_4$ alkyl group such as methyl or ethyl.

In summary therefore, the new anti-tumour compounds of the present invention are compounds of the formula:

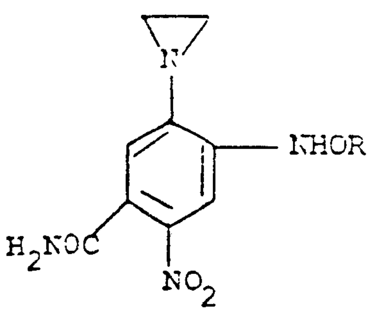

where R is H or an acyl group, e.g., a carboxylic acyl group of up to 6 carbon atoms particularly an acetyl group or R is a phosphate or sulphate group.

The hydroxylamines of the present invention can be prepared by reduction of the corresponding 4-nitro compound, that is CB 1954. We have found that the most satisfactory method of selective reduction of the 4-nitro group is to use zinc dust and ammonium acetate in acetone in accordance with the method of Jarman et al, Biomed. Mass. Spectrom. 1983, 10, 1983. The 4-hydroxylamine obtained by the selective reduction method mentioned above can then be converted into its ester or ether derivatives by methods known per se using the appropriate esterifying or etherifying agent.

An alternative method of preparing the hydroxylamines of the present invention is, of course, by selective reduction of CB 1954 using the enzyme EC 1.6.99.2 and isolating the resulting 4-hydroxylamino compound.

Further aspects of the present invention comprise methods of controlling neoplastic tissue growth in humans or animals by converting cytotoxic pro-drugs in vivo into cytotoxic drugs by the use of the enzyme EC 1.6.99.2 in methods involving initially the administration of the enzyme to a host, the enzyme optionally being conjugated with an antibody that will bind to a tumour associated antigen, and then subsequently administering a cytotoxic pro-drug, e.g. CB 1954, for example by intravenous injection at a rate of about 0.2-1.6 mg/Kg body weight corresponding to a clinical dosage of about 12.5 to 100 mg. A specific dosage regime involves injection of 25 mg followed by a further 12.5 mg every third day.

Further aspects of the present invention comprise pharmaceutical compositions comprising the hydroxylamines of the present invention, or their ether and ester derivatives, in a formulation suitable for parenteral administration. Such compositions will normally be prepared in a form suitable for intravenous administration.

The invention also extends to methods of treatment for controlling neoplastic tissue growth by administering to a human in need of such treatment effective amounts e.g. by the intravenous route, of CB 1954 and EC 1.6.99.2.

In an alternative embodiment, the invention provides a prodrug/enzyme system for use in controlling neoplastic tissue growth in humans comprising (1) an amount of 5-(aziridin-1-yl)-2,4-dinitrobenzamide in a form suitable for parenteral administration in association with (2) an amount of the enzyme EC 1.6.99.2 such that the 5-(aziridin-1-yl)-2,4-dinitrobenzamide is converted in vivo into 5-(aziridin-1-yl)-4-hydroxylamino-2-nitrobenzamide.

## Description of the Drawings

Figure 1 illustrates the results of cytotoxicity tests described in Example 2.

Figure 2A and 2B illustrate the results of comparative tests described in Examples 3 and 4.

Figures 3 and 4 illustrate the results of cytotoxicity tests described in Example 6.

The present invention is further illustrated by the following Examples.

## EXAMPLE 1

This describes the isolation of the EC 1.6.99.2 enzyme from Walker tumour cells.

Walker 256 cells growing in stirred suspension culture were resuspended at 2 x $10^8$ cells/ml in ice-cold PBS + 1% aprotinin and disrupted by sonication. The pale yellow supernatant (5 ml) obtained after centrifugation (60,000 g for 1 hour at 4°C), was injected onto a TSK G3000 SWG (21.5 x 600 mm) gel filtration column, eluted at ambient temperature (3 ml/min) with 0.01 M $NaH_2PO_4$/$Na_2HPO_4$ pH 7, and 3 ml fractions collected. Reductase activity was detected by the loss of CB 1954 (determined by HPLC) in the presence of the enzyme. A solution of CB 1954 (300 µM) and NADH ( 5mM) in PBS was incubated with fractions from the column (50 µl), the mixture incubated at 37°C for 1

hour, then aliquots (10 µl) injected on a reverse phase HPLC column and eluted isocratically (1 ml/min) with 0.1M $NH_4OAc$ (pH 7) in 25% methanol. The eluate was continually monitored at 325 nm, CB 1954 eluting as a single resolved peak after about 7 minutes. Fractions containing reductase activity were pooled and concentrated by centrifugation through a polysulphone membrane (Millipore Ultrafree 30,000 N.M.W.L.) and the concentrate (2 ml) reinjected onto the above column. Active fractions were again pooled and concentrated and injected (2 ml) onto a TSK DEAE-5PW (7.5 x 75 mm) anion exchange column, eluted with a NaCl gradient (0-0.5 M linear over 40 minutes) in $NaH_2PO_4/NaHPO_4$, pH 7 (1 ml/min) and 0.5 ml fractions collected. Active fractions were pooled, concentrated and reinjected and the purified protein stored at -20°C. Protein purity (>95%) was confirmed by PAGE as a single band of 33.5 Kd. Yield was about 10 mg per $10^{10}$ cells. The protein was quantified by its absorbance at 450 nm given that 1 mg per ml gives an absorbance of 0.125 (10 mm pathlength).

The yellow enzyme was heated at 56° for 20 minutes and the separated flavin cofactor removed from the pure protein by ultrafiltration. The pure protein having a molecular weight of about 33.5 Kd was then subjected to amino acid composition analysis when the following results were obtained.

| | Conc. nmoles/ml |
|---|---|
| ASP | 351.696 |
| THR | 178.395 |
| SER | 306.753 |
| GLU | 652.701 |
| PRO | 330.577 |
| GLY | 405.314 |
| ALA | 421.862 |
| CYS* | 18.594 |
| VAL | 348.137 |
| MET* | 91.248 |
| ILE | 266.834 |
| LEU | 544.655 |
| NLE | |
| TYR | 224.328 |
| PHE | 324.181 |
| HIS | 110.181 |
| LYS | 429.624 |
| ARG | 221.772 |

* CYS AND MET PARTIALLY DESTROYED BY HYDROLYSIS

## EXAMPLE 2

Cytotoxicity of products of enzymic reduction of CB 1954

A mixture of (U-$^3$H) CB 1954 (30 µM; 140 µCi/mMol), NADH (500 µM) and enzyme (50 ng) were incubated in PBS (1 ml) at 37°C. At various times a sample (200 µl) was removed and injected onto a ODS-5 reverse phase HPLC column and eluted (1 ml/min) with a methanol gradient (0-30% linear over 30 minutes, 30-100% linear over 10 minutes) in 0.1 M $Na_2HPO_4/NaH_2PO_4$. Samples (1 ml) were collected and the tritium activity of each determined by scintillation counting. The results are shown in Figure 1, A, at 0 minutes; B, at 90 minutes, C, at 180 minutes. To determine if the products identified from the above were also cytotoxic CB 1954 (200 µM), NADH (5 mM) and enzyme (100 ng) were incubated in PBS (600 µl) at 37°C. After 2 hours an aliquot (500 µl) was injected onto the HPLC column and eluted as described above. Samples (1 ml) were collected, individually sterilised by passage through 0.2 µM filters and added (500 µl) to Walker 256 cells (10 ml/2 x $10^5$ per ml) which, after a 2 hour incubation at 37°C were assayed out for colony forming ability. The results are plotted as survival fraction against column fraction number as shown in Figure 1D. The cytotoxicity towards Chinese hamster cells of the synthesised hydroxylamino derivatives of CB 1954 was confirmed by a similar method to the above. A sample (200 µl) of a 200 µM solution of an equal mixture of the 2 and the 4-hydroxylamines was injected onto the HPLC column and the cytotoxicity of the resulting fractions determined as above and the result shown in Figure 1E.

## EXAMPLE 3

This shows formation of crosslinks in the DNA of Chinese hamster cells treated with CB 1954 and a bioactivating enzyme mixture. Cells were labelled by growth for 24 hours in the presence of $^{14}$C thymidine (50 µCi/ml; 50 mCi/mM) followed by 2 hours in the absence of label, treated with CB 1954 (10 µM) and NADH (100 µM) and various quantities of the enzyme for 18 hours, washed (PBS) irradiated (5 Gy) and their DNA analysed by sucrose gradient sedimentation. The results are shown in Figure 2A where the symbols indicate (○) no enzyme; (♦) 1 µg/ml; (□) 10 µg/ml; (▲) 100 µg/ml reductase enzyme.

## EXAMPLE 4

This shows the formation of crosslinks in the DNA of Chinese hamster cells treated with 5-(aziridin-1-yl)-4-hydroxylamino-2-nitrobenzamide. Cells were labelled as in Example 3, treated with the 4-hydroxylamine for 2 hours, washed with PBS, irradiated (5 Gy) and subjected to alkaline sucrose gradient sedimentation as described in Example 3. The results are shown in Figure 2B where the symbols indicate (○) untreated; (▲) 5 µM; (□) 20 µM hydroxylamine. Sedimentation was from left to right.

## EXAMPLE 5

Synthesis of 5-(Aziridin-1-yl)-4-hydrolylamino-2-nitrobenzamide (3)

$^1$H-NMR spectra (250 MHz) were obtained using a Bruker AC250 instrument. For thin-layer chromatography glass plates (5 x 20 cm) coated with silica gel 60F-254 (Merck) ((0.25 mm) were used. Details of column chromatography are given below. Melting points were determined with a Kofler hot-stage.

To a vigorously stirred solution of 5-(aziridin--1-yl)-2,4-dinitrobenzamide (1, 1,008g, 4 mmol) in acetone (40 ml) was added Zn dust (500 mg) and $NH_4OAc$ (500 mg) with repeat additions of Zn and $NH_4OAc$ after 2, 4 and 6 minutes. After 8 minutes, solid was removed by filtration. The orange-red filtrate was diluted with $CH_2Cl_2$ (60 mL) and the solution was applied to a column (25 x 3 cm) of silica gel comprising a layer of coarser silica (10 g, Art. 7734) to allow rapid flow of solvent through the sintered glass frit, overlaid with finer grade silica (70 g, Art. 9385). The column was eluted under slight pressure ($N_2$), at a flow rate of ca. 20 mL. min. $^{-1}$, with acetone-$CH_2Cl_2$ (1:1). After a forerun (140 mL), 1 (770 mg) was eluted (160 mL), then 5-(aziridin-1-yl)-2-hydroxylamino-4-nitrobenzamide (2) (140 mL), a mixture of 2 and 5-(aziridin-1-yl)-4-hydroxylamino-2-nitrobenzamide (3) (80 mL) then 3 (240 mL). The fraction containing 2 was concentrated to ca. 5 mL whereupon 2 (17 mg, 7.8% based on 1 consumed), separated as garnet rhombs which darkened without melting above 200°C. $^1$H NMR ($Me_2SO$-$d_6$)
δ 2.20 (s, 4H, aziridinyl H), 7.42 (s, 1H, H-6), 7.63 (s, 1H, H-3) 7.71 (s, 1H, other of amide NH), 8.23 (s, 1H, one of amide NH), 8.85 (d, 1H, OH, J 2.2 Hz), 9.12 (poorly resolved d, 1H, NHOH). Anal. ($C_9H_{10}N_4O_4$) C, H, N. The fraction containing 3 gave on concentration to ca. 20 mL, 3 (29 mg, 13% based on 1 consumed) as minute yellow needles which darkened above 200°C without melting. $^1$H NMR ($Me_2SO$-$d_6$)
δ 2.14 (s, 4H, aziridinyl H), 6.94 (s, 1H, H-6), 7.43 (s, 1H, H-3), 7.45 (s, 1H, other of amide NH), 7.89 (s, 1H, one of ammide NH), 8.57 (br s, 1H, NHOH), 8.88 (d, 1H, OH, J 1.5 Hz). Anal. C, H. N.

$^1$H - NMR Spectra of Isomeric Aminonitro Derivatives (4 and 5).

The aminonitro derivatives were prepared by the procedure of Jarman et al, Biochem. Pharmacol. 1976, 2475. NMR ($Me_2SO$-$d_6$): 2-amino-5-(aziridin-1-yl)-4-nitrobenzamide (4) δ 2.11 (s, 4H, aziridinyl H), 6.46 (s, 2H, $NH_2$), 7.24 (s, 1H, H-6), 7.32 (s, 1H, H-3), 7.46 (s, 1H, other of amide NH), 8.06 (s, 1H, one of amide NH); 4-amino-5-(aziridin-1-yl)-2-nitrobenzamide (5) 2.19 (s, 4H, aziridinyl H), 5.33 (s, 2H, $NH_2$), 6.6 (br s, 1H, other of amide NH), 6.98 (s, 1H, H-6), 7.2 (br s, 1H, one of amide NH), 7.22 (s, 1H, H-3).

EXAMPLE 6

THE EFFECT OF CB 1954 AND ITS 4-HYDROXYLAMINO DERIVATIVE ON THE SURVIVAL OF VARIOUS CELL TYPES

Suspensions of various test cells were exposed to μM concentrations of the agents CB 1954 and its 4-hydroxylamino derivatives, (formula I compound where R is H) for two hours, after which the agent was removed and the viability of the cells assayed by colony-forming ability. The results are shown in Figure 3 for CB 1954 and Figure 4 for its 4-hydroxylamino derivative.

It can be seen that the response to CB 1954 (a) falls into two main groups. There is firstly the sensitive cell type, as previously exemplified by the crosslink-sensitive Walker tumour cell (WS) and a crosslink resistant subline thereof (WR), whose number now includes HSN, BL8, and JB1, all rat tumour cells. Secondly, there is the resistant group, originally exemplified by chinese hamster V79 cells, but now including L1210 (a mouse leukaemia), HeLa (a well established human tumour cell line), HFL (a normal human cell), MAWI (a human colon carcinoma cell), and CEM (a human lymohoma cell). Hep-G2, a human hepatoma cell known to be high in DT diaphorase, is somewhat more sensitive than the rest of this group.

The response of several of these test cells to the 4-hydroxylamino derivative of CB 1954 is shown in Figure 4 where they are seen to be equally as sensitive as the WR to both CB 1954 and the hydroxylamine. By virtue of their special crosslink sensitivity, the WS cells are (as with CB 1954) somewhat more sensitive again.

**Claims**

1. A compound of the general formula:

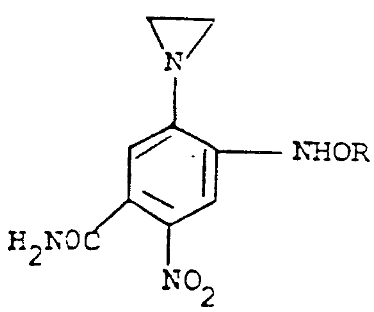

wherein R is H, an acyl group or a hydrocarbyl group containing up to 6 carbon atoms.

2. A compound according to claim 1 wherein R is H.

3. A compound according to claim 1 wherein R is a carboxylic acyl group containing up to 6 carbon atoms.

4. A process for the preparation of a compound of formula I as defined in claim 1 wherein R is H which comprises subjecting

5-(aziridin-1-yl)-2,4-dinitrobenzamide to the action of the enzyme EC 1.6.99.2 and isolating the resulting 5-(aziridin-1-yl)-4-hydroxylamino-2-nitrobenzamide.

5. A process for the preparation of a compound of formula I as defined in claim 1 wherein R is H which comprises selective reduction of 5-(aziridin-1-yl)-2,4-dinitrobenzamide with zinc and ammonium acetate.

6. A process according to claim 4 or 5 followed by the step of esterifying or etherifying the 4-hydroxylamino group to give the corresponding formula I compound wherein R is an acyl or hydrocarbyl group.

7. A pharmaceutical composition comprising a compound according to any one of claims 1 to 3 together with a pharmaceutically acceptable carrier or diluent.

8. A composition according to claim 7 suitable for parenteral administration.

9. A composition according to claim 8 suitable for intravenous administration.

10. A compound according to any one of claims 1 to 3 for use in a method of treatment of the human or animal body by surgery or therapy or in a diagnostic method practised on the human or animal body.

11. A compound according to any one of claims 1 to 3 for use in a method for treatment of the human or animal body to control neoplastic tissue growth.

12. A method of treatment of the human or animal body to control neoplastic tissue growth which comprises administering to a human or animal host in need of such treatment an effective amount of a compound according to any one of claims 1 to 3.

13. 5-(Aziridin-1-yl)-2,4-dinitrobenzamide for use in association with the enzyme EC 1.6.99.2 in a method for the treatment of the human body to control neoplastic tissue growth.

14. A method of treatment of the human body to control neoplastic tissie growth which comprises administering to a human host in need of such treatment effective amounts of 5-(aziridin-1-yl)-2,4-dinitrobenzamide with the enzyme EC 1.6.99.2.

15. A prodrug/enzyme system for use in controlling neoplastic tissue growth in humans comprising (1) an amount of 5-(aziridin-1-yl)-2,4-dinitrobenzamide in a form suitable for parenteral administration in association with (2) an amount of the enzyme EC 1.6.99.2 such that the 5-(aziridin-1-yl)-2,4-dinitrobenzamide is converted in vivo into 5-(aziridin-1-yl)-4-hydroxylamino-2-nitrobenzamide.

16. An antibody to the enzyme EC 1.6.99.2.

Fig. 1.

Cytotoxicity of enzyme-produced products of CB 1954 and of 5-(aziridin-1-yl)-4-hydroxylamino-2-nitrobenzamide.

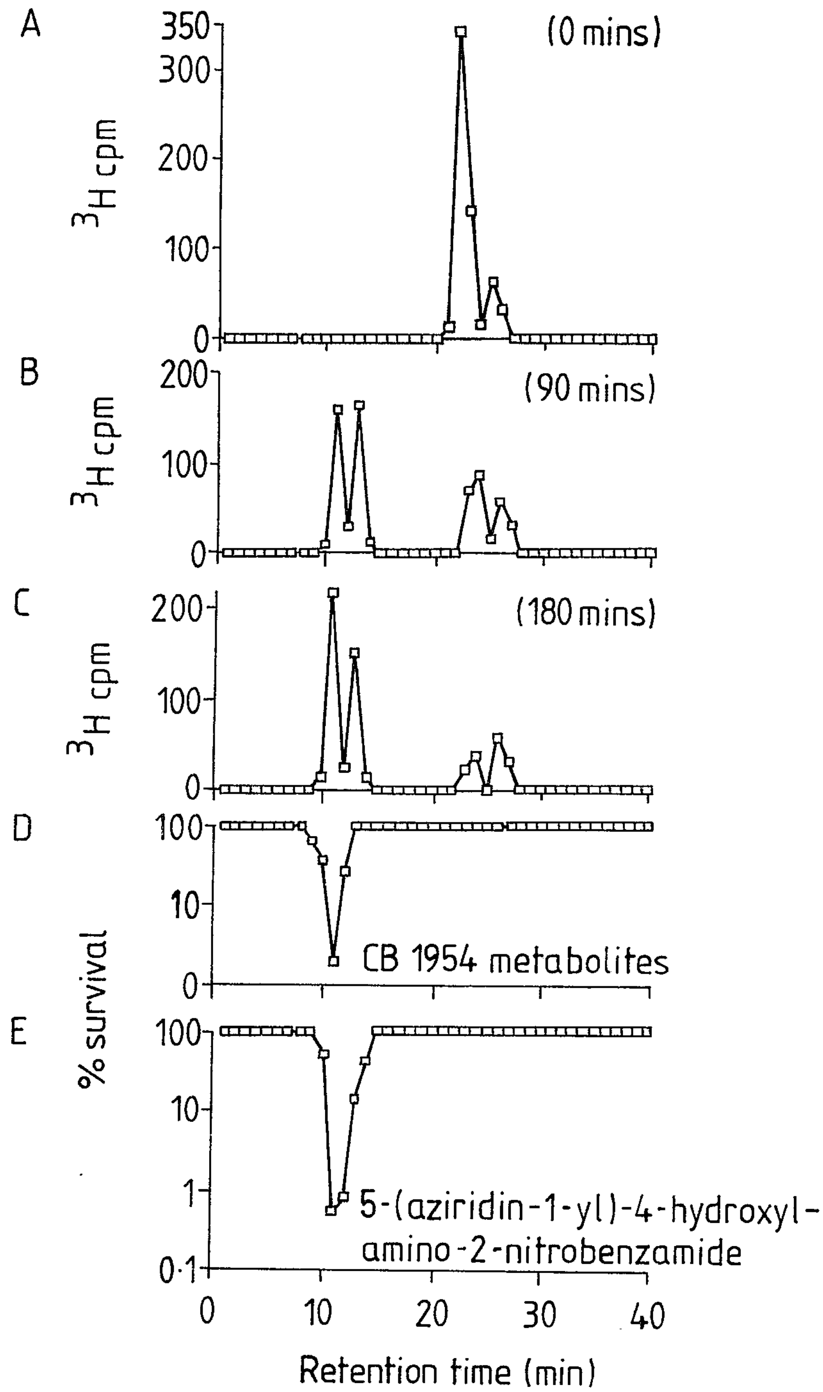

Fig.2A.

Crosslinking of chinese hamster cell DNA using a mixture of Walker cell-derived nitroreductase and CB 1954

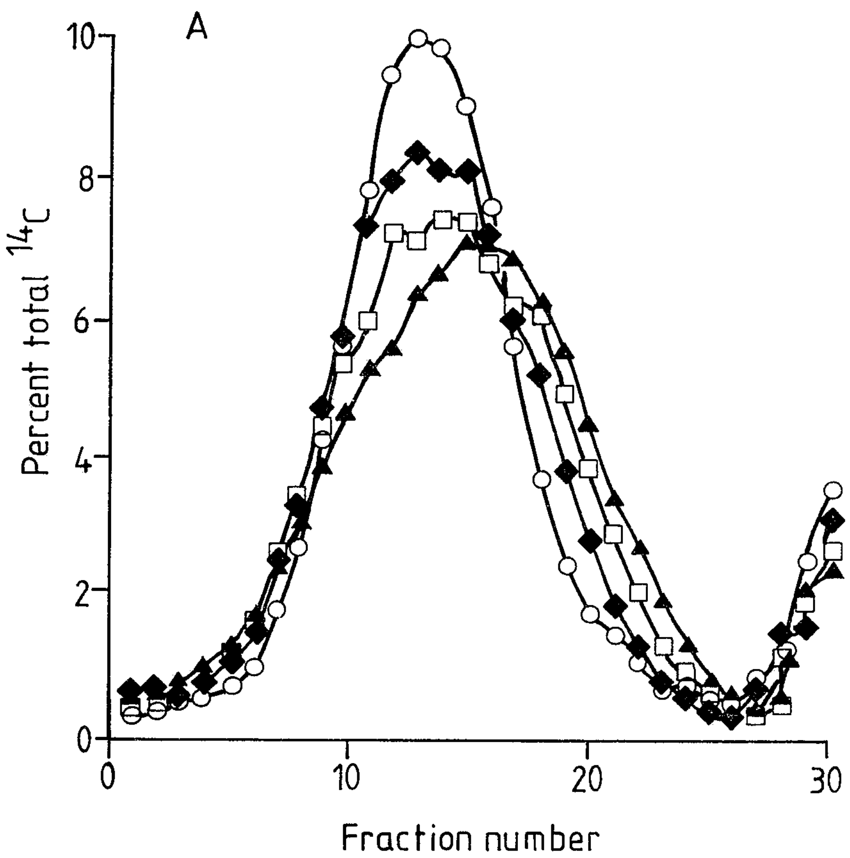


Fig.2B.

Crosslinking of chinese hamster cell DNA with 5-(Aziridin-1-yl)-4-hydroxylamino-2-nitrobenzamide

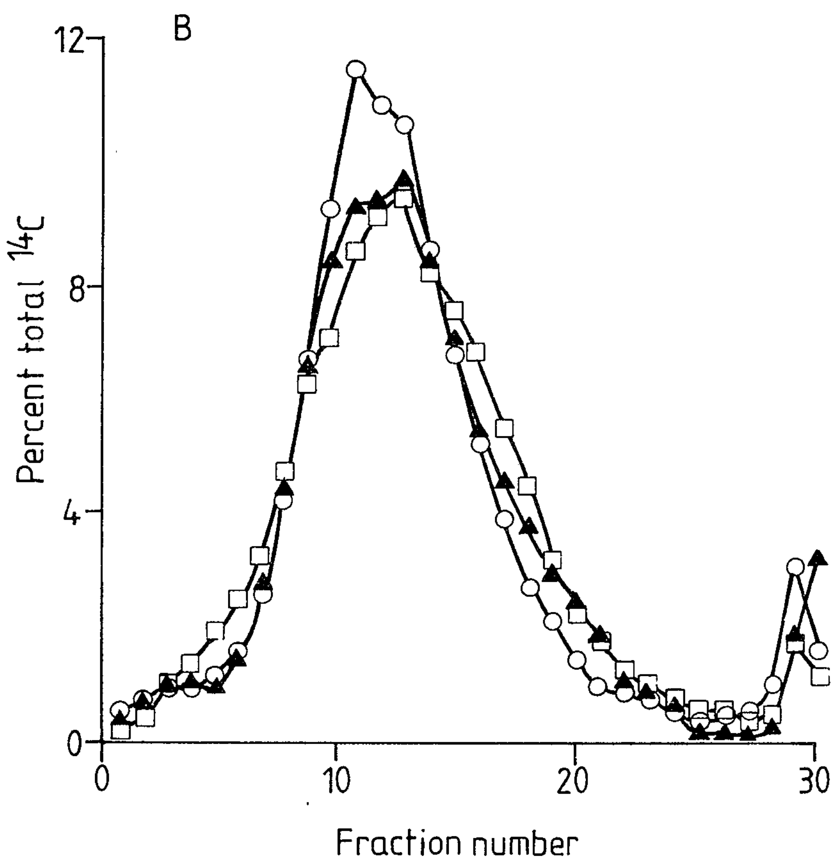

Fig.3.

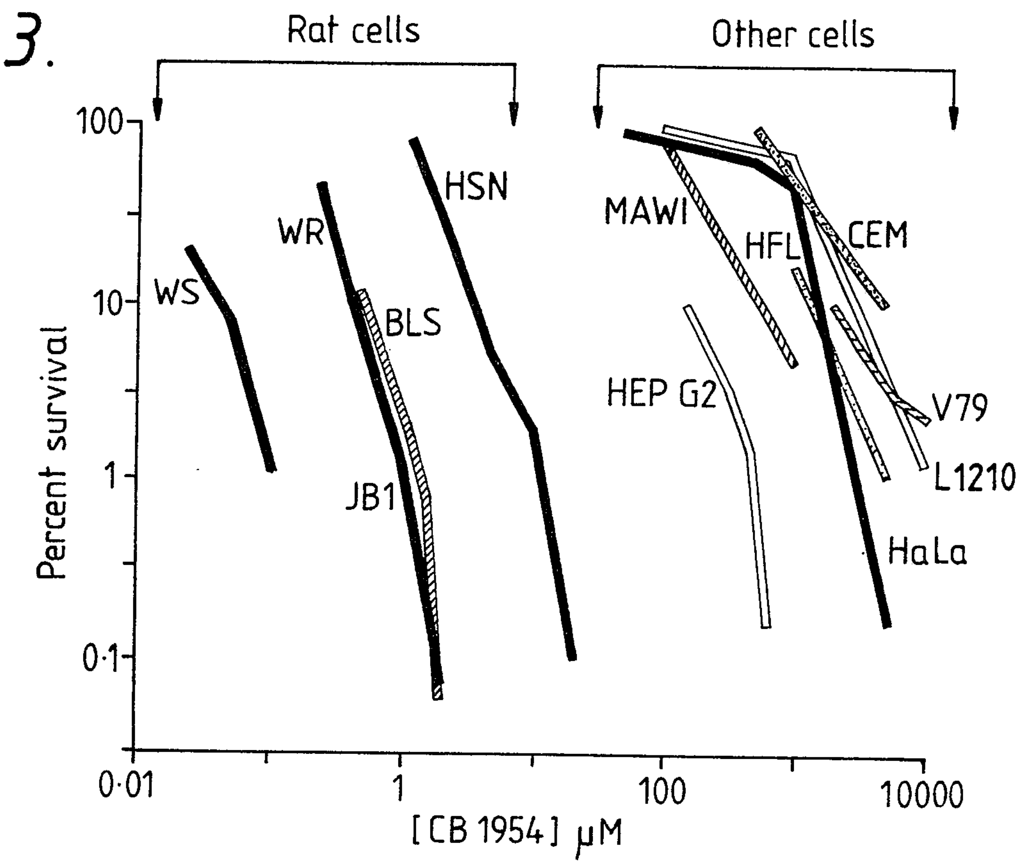
Rat cells
Other cells
100
10
1
0·1
Percent survival
WS
WR
BLS
JB1
HSN
MAWI
HFL
CEM
HEP G2
V79
L1210
HaLa
0·01
1
100
10000
[CB 1954] μM

Fig.4.

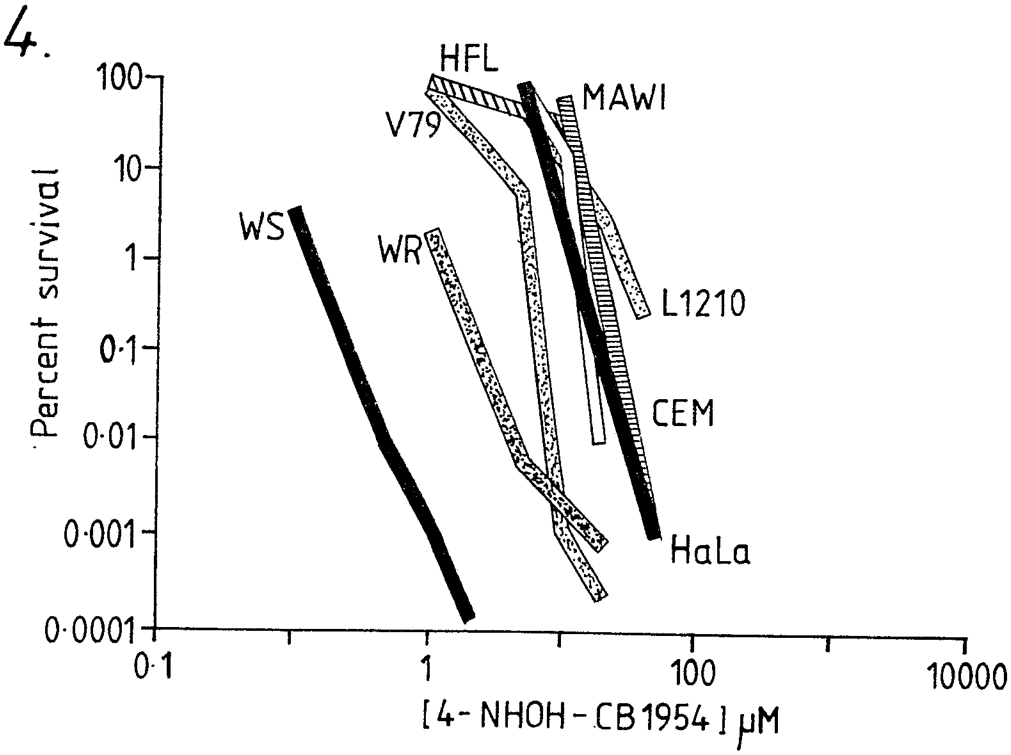
100
10
1
0·1
0·01
0·001
0·0001
Percent survival
HFL
MAWI
V79
WS
WR
L1210
CEM
HaLa
0·1
1
100
10000
[4-NHOH-CB1954] μM

European Patent Office

# PARTIAL EUROPEAN SEARCH REPORT

which under Rule 45 of the European Patent Convention shall be considered, for the purposes of subsequent proceedings, as the European search report

Application number: EP 89301678.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X | THE JOURNAL OF BIOLOGICAL CHEMISTRY, vol. 261, no. 31, November 1986<br>J.A. ROBERTSON et al. "NAD(P)H: Menadione oxidoreductase" pages 15794-15799<br>* Page 15797, right-hand column, paragraph 4 from the bottom *<br>-- | 16 | C 07 D 203/14<br>C 12 P 17/10<br>A 61 K 31/395<br>A 61 K 37/50<br>C 07 K 15/00 |
| A | US - A - 4 318 980 (BOGULASKI et al.)<br>* Claims 1,24,37 *<br>-- | | |
| A | CESARE FERRI "Reaktionen der organischen Synthese", 1978<br>GEORG THIEME VERLAG, Stuttgart page 112<br>* Page 112, 1.3.3.7.1.(4) *<br>-- | 5 | |

**TECHNICAL FIELDS SEARCHED (Int. Cl.4)**

C 07 D 203/00
C 12 P 17/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-11,13,15,16
Claims searched incompletely: –
Claims not searched: 12,14
Reason for the limitation of the search:

(Method for treatment of the human or animal body by therapie, Article 52(4) EPC)

| Place of search | Date of completion of the search | Examiner |
|---|---|---|
| VIENNA | 16-05-1989 | KÖRBER |

**CATEGORY OF CITED DOCUMENTS**

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

European Patent Office

# PARTIAL EUROPEAN SEARCH REPORT

Application number

EP 89301678.2

| DOCUMENTS CONSIDERED TO BE RELEVANT | | | CLASSIFICATION OF THE APPLICATION (Int. Cl.4) |
|---|---|---|---|
| Category | Citation of document with indication, where appropriate, of relevant passages | Relevant to claim | |
| P,X | BIOCHEMICAL PHARMACOLOGY, vol. 37, no. 24, December 1988, Oxford<br>R.J. KNOX et al. "The nitroreduc--tase enzyme in Walker cells that activates 5-(aziridin-1-yl)-2,4--dinitrobenzamide (CB 1954) to 5-(aziridin-1-yl)-4-hydroxyl--amino-2-nitrobenzamide is a form of NADP(H) dehydrogenase (quinone) (EC 1.6.99.2)"<br>pages 4671-4677<br>* Page 4676, right-hand column, lines 40-61 *<br>---- | 1,2,4, 13,15, 16 | |
| | | | TECHNICAL FIELDS SEARCHED (Int. Cl.4) |

EPO Form 1505.3 06.78